# EUROPEAN PATENT SPECIFICATION

(11) **EP 1 801 226 B1**
(45) Date of publication and mention of the grant of the patent: **18.05.2011**
(21) Application number: 06077261.3
(22) Date of filing: 22.01.1992
(51) Int. Cl.: C12P 7/64, A23D 9/00, A23C 11/04, A61K 31/20, A61K 8/92, A23L 1/30

(54) **Arachidonic acid and methods for the production and use thereof**
Arachidonsäure und Verfahren zu ihrer Herstellung und Verwendung
Acide arachidonique et son procédé de production et d'utilisation

(30) Priority: 24.01.1991 US 645454
(43) Date of publication of application: 27.06.2007
(62) Divisional of application: 05077139.3
(73) Proprietor: MARTEK BIOSCIENCES CORPORATION, Columbia, MD 21045 (US)
(72) Inventor: Kyle, David J., Catonsville, MD 21228 (US)
(74) Representative: Mercer, Christopher Paul

(56) References cited:
- EP-A- 0 223 960
- EP-A- 0 276 541
- JP-A- 1 038 007
- JP-A- 1 304 892
- JP-A- 2 086 789
- JP-A- 01 196 255
- ARTEMIS P. SIMOPOULOS: "Summary of the NATO advanced research workshop on dietary w3 and w6 fatty acids: Biological effects and nutrtional essentiality" THE JOURNAL OF NUTRITION, vol. 119, 1989, pages 521-528, XP002445442
- YOSHIFUMI SHINMEN ET AL.: "Production of arachidonic acid by Mortierella fungi" APPLIED MICROBIOLOGY AND BIOTECHNOLOGY, vol. 31, 1989, pages 11-16, XP001027373
- NAGAO TOTANI ET AL.: "A simple method for production of arachidonic acid by Mortierella alpina" APPL. MICROBIOL. BIOTECHNOL., vol. 28, 1988, pages 135-137, XP008078083
- WICHIEN YONGMANITCHAI ET AL.: "Omega-3 fatty acids: alternative sources of production" PROCESS BIOCHEMISTRY, August 1989 (1989-08), pages 117-125, XP003004328
- S.R. GANDHI ET AL.: "Production of the polyunsaturated fatty acids arachidonic acid and eicosapentaenoic acid by the fungus Pythium ultimum" JOURNAL OF GENERAL MICROBIOLOGY, vol. 137, 1991, pages 1825-1830, XP000919111
- PRAMOD K. BAJPAI ET AL.: "Production of arachidonic acid by Mortierella alpina ATCC 32222" JOURNAL OF INDUSTRIAL MICROBIOLOGY, vol. 8, 1991, pages 179-186, XP002445443
- PRAMOD K. BAJPAI ET AL.: "Arachidonic acid production by fungi" APPLIED AND ENVIRONMENTAL MICROBIOLOGY, vol. 57, no. 4, April 1991 (1991-04), pages 1255-1258, XP002445444
- PRATIMA BAJPAI ET AL.: "Arachidonic acid production by microorganisms" BIOTECHNOLOGY AND APPLIED BIOCHEMISTRY, vol. 15, 1992, pages 1-10, XP008081555
- YAMADA H ET AL: "BIOTECHNOLOGICAL PROCESSES FOR PRODUCTION OF POLY-UNSATURATED FATTY ACIDS", JOURNAL OF DISPERSION SCIENCE AND TECHNOLOGY, TAYLOR AND FRANCIS GROUP, NEW YORK, NY, US LNKD- DOI:10.1080/01932698908943188, vol. 10, no. 4/05, 1 January 1989 (1989-01-01), pages 561-579, XP000884958, ISSN: 0193-2691

## Description

This invention relates to infant formula containing arachidonic acid.

Arachidonic acid (ARA) is a long chain polyunsaturated fatty acid (PUFA) of the omega-6 class (5,8,11,14-eicosatetraenoic acid, i.e., 20:4). ARA is the most abundant C₂₀ PUFA in the human body. It is particularly prevalent in organ, muscle and blood tissues, serving a major role as a structural lipid associated predominantly with phospholipids in blood, liver, muscle and other major organ systems. In addition to its primary role as a structural lipid, ARA also is the direct precursor for a number of circulating eicosenoids such as prostaglandin E₂ (PGE₂), prostacyclin I₂ (PGI₂), thromboxane A₂ (TₓA₂), and leukotirenes B₄ (LTB₄) and C₄ (LTC₄). These eicosenoids exhibit regulatory effects on lipoprotein metabolism, blood rheology, vascular tone, leucocyte function and platelet activation.

Despite its importance to human metabolism, ARA cannot be synthesized in humans de novo. ARA is synthesized by the elongation and desaturation of linoleic acid (LOA), an essential fatty acid. This process requires the presence of the enzyme Δ6-desaturase, an enzyme present in the human body in low levels, Burre et al., Lipids, 25:354-356 (1990). Accordingly, most ARA must be provided in the diet, and this is especially important during times of very rapid body growth, such as infancy.

During the first year of its life, an infant can double or triple its weight. Consequently, elevated levels of dietary ARA are required. To satisfy this increased demand, human breast milk contains high levels of ARA. Sanders et al., Am. J. Clin. Nutr., 31:805-813 (1970). ARA is the most prevalent C₂₀ PUFA in breast milk. Of those mothers, especially vegetarians, who do breast-feed their infants, many would benefit from additional dietary ARA. However, many mothers do not breast feed their infants, or do not breast feed for the entire period of rapid infant growth, choosing instead to utilize an infant formula.

No commercial infant formulas known to Applicant contain ARA. U.S. Patent No. 4,670,285 (Clandinin et al.), discloses the infant's requirement for fatty acids including ARA. To provide these fatty acids, Clandinin et al. suggest a blend of egg yolk, fish oil or red blood cell phospholipids and vegetable oils as the fat component of a proposed infant formula. However, fish oil contains high quantities of eicosapentaneoic acid (EPA). EPA is known to depress ARA synthesis in infants. Carlson, et al., INFORM, 1:306 (1990). Thus, it would be desirable to be able to provide ARA without also providing additional. EPA. Furthermore, egg yolks contain a relatively low concentration of ARA, such that Clandinin et al.'s mixture is not economically viable.

Because ARA is present in animal, but not vegetable, oils, its production in commercial quantities has remained a desirable, but elusive, goal. Shinmen, et al., Microbiol. Biotech. 31:11-16 (1989), have reported the production of ARA by an isolated fungus, *Mortierella alpina,* using conventional stirred tank fermentation. (See also Japanese Patent 1,215,245 to Shinmen et al.). After culturing, the organisms are harvested, dried and their lipids extracted from the fungal biomass with an organic solvent and the lipids chemically (covalently) modified. For example, the lipid mixture is hydrolyzed or converted to ethyl esters and then combined with cyclodextrin prior to use as a dietary supplement. Shinmen et al. do not disclose or suggest the administration of unmodified microbial oils.

*Porphyridium cruentum,* a red microalgae, can be grown in ponds in large quantities and has a lipid content which can contain up to 40% ARA. Ahern, et al. Biotech. Bioeng. 25;1057-1070 (1983). Unfortunately, the ARA is primarily associated with galactolipids, a complex polar lipid not present in breast milk. Thus, not only is the total usable ARA produced a fraction of one percent of the biomass, but the form of the ARA is not suitable for use as an additive to infant formula without further modification.

U.S. Patent No. 4,870,011 (Suzuki et al.) discloses a method for obtaining lipids such as γ-linolenic acid from fungi of the genus Mortierella. The γ-linolenic acid is purified from the mixture of lipids contained in the fungi.

DE 3603000A1 (Milupa) discloses a highly polyunsaturated acid fat mixture and its use as the fat component of an infant formula. The fat mixture has a high content of ARA and docosahexanoic (DHA) acids in a ratio of 2.5:1 respectively, as well as a high content of cholesterol. Sources of the fatty acids are listed as being certain types of macroalgae, fish oils, organ fats from beef and pork or highly refined egg yolk oil. A source of the DHA and ARA is said to be macroalgae of the phaecophyte and rhodophyte types. There is no suggestion to use any microbes as a source of oil. Algal and fish oils also typically include EPA which depresses ARA synthesis in vivo. Additionally, highly refined egg yolk oil is not an economical source of ARA. Moreover, there is no disclosure therein of an ARA-concentrated additive for supplementing pre-existing infant formula.

JP 01-196255 describes using fatty acids derived from Mortierella in infant formula. In Example 4, fermentation of M. elongata is carried out to form an ethyl esterified lipid which includes EPA and ARA in a ratio of 1:2.

Accordingly, there remains a need for an economical, commercially feasible method of producing ARA, preferably without concomitant production of EPA. It is an object of the present invention to satisfy that need.

it is a further object of the invention to provide infant formula such that the ARA levels in the formula approximate those levels in human breast milk.

### Summary of the Invention

This invention relates to infant formula containing arachidonic acid containing fungal oil (ARASCO). The oil can be referred to as a single cell oil. Fungi are cultivated under oil-producing conditions, harvested and the oil extracted and recovered. The oil, without further chemical modification, can be used directly to provide supplemental ARA. Advantages of the oil used in the invention include its ease of production, and high purity, and lack of detectable amounts of EPA.

In particular, the present invention provides infant formula comprising between 50 to 1000 mg per liter of infant formula of an arachidonic acid (ARA) containing fungal oil (ARASCO), wherein
(i) EPA is present in the oil in less than one fifth of the amount of ARA in the oil,
(ii) the ARA content in the oil is from 10 to 50% of the fatty acids in the oil;
(iii) the oil is predominantly triglyceride and unmodified; and
(iv) the ARA : EPA ratio in the infant formula is at least 5:1.

### Detailed Description of the Preferred Embodiment of the Invention

The present invention succeeds in providing an economical source of arachidonic acid (ARA). In the infant formula of the present invention, EPA is present in less than about one fifth of the amount of ARA in the oil. This oil, a single cell oil, is included in the infant formula, in an unmodified form. As used herein "unmodified" means that the chemical properties of the fatty acids, or the oils themselves, have not been covalently altered. Thus, for example, a temporary modification to the ARASCO or ARA which could be reversed following uptake of the oil would not be beyond the scope of this invention.

**Table 1. Fatty Acid Composition of Several Fungal Species.**

| | Fatty Acid | | | | | | | | total |
|---|---|---|---|---|---|---|---|---|---|
| Species | 14:0 | 16:0 | 16.1 | 18:1 | 18:2 | 18:3 | 20:4 | 20:5 | fat |
| *Mortierella alpina* | -- | 8.2 | -- | 33.5 | 16.3 | 23.3 | 13.0 | -- | 3.0 |
| *Mortierella elongata* | 2.0 | 13.2 | -- | 26.6 | 11.9 | 13.2 | 13.8 | 2.4 | 4.0 |
| *Mortierella isabellina* | 0.3 | 15.7 | 0.8 | 55.8 | 11.1 | 9.0 | -- | -- | 7.3 |
| *Saprolegnia parasitica* | 7.4 | 19.1 | 1.9 | 6.3 | 24.5 | 12.5 | 10.5 | 10.5 | 9.3 |
| *Pythium catenulatum* | 6.5 | 9.9 | 10.3 | 21.2 | 18.5 | 3.5 | 13.4 | 10. 9 | 5.0 |
| *Pythium coloratum* | 13.6 | 9.9 | -- | 14.7 | 10.9 | 2.5 | 24.3 | 21.7 | 2. 2 |
| *Pythium gracile* | 14.7 | 9.1 | 2.2 | 14.8 | 12.6 | 3.6 | 22.1 | 5.7 | 4.5 |
| *Pythium irregulare* | 10.3 | 15.4 | 6.9 | 12.3 | 21.0 | 3.9 | 10.6 | 12.4 | 11.9 |
| *Pythium ultimum* | 9.5 | 16.7 | 10.5 | 17.1 | 20.7 | 1.3 | 9.0 | 6.9 | 13.3 |
| *Pythium insidiosum* | 9. 5 | 11.4 | 12.1 | 1.0 | 8.3 | 9.3 | 31.9 | -- | 2.8 |

Of those fungal species which previously have had their fatty acids characterized, it has been found that most do not make ARA. Weete, J.D., Fungal Lipid Biochemistry, Plenum Press, N.Y. (1974). Of those species which do make ARA, many, including all previously characterized *Pythium* species, produce significant quantities of eicosapentaenoic acid (EPA) in addition to ARA. Table 1 sets forth the fatty acid profile of *P. insidiosum* as well as the fatty acid profile of other species of fungi. Unexpectedly, it has been found that *P. insidiosum* produces ARA without concomitant production of EPA. As with fish oils, high EPA levels in dietary supplements result in a depression of the ability to form ARA from dietary linoleic acid (LOA). Accordingly, while those fungal species producing both ARA and EPA can be utilized in the process described herein, it is preferable to use species which do not produce significant quantities of EPA. Such preferred species include *Pythium insidiosum* and *Mortierella alpina.* Both species are available commercially and are on deposit with the American Type Culture Collective in Rockville, Maryland, having accession numbers 28251 and 42430, respectively. Throughout this disclosure, unless otherwise expressly stated, *P. insidiosum* will be the representative fungal species.

One of the significant problems which an embodiment of the present invention overcomes, is the depression of ARA biosynthesis in infants caused by the presence of enhanced dietary levels of EPA. This problem can be corrected by providing ARA in infant formula at levels substantially similar to those found in human breast milk. Typically in human breast milk, the ratio of ARA:EPA is about 20:1 respectively. The present invention specifically contemplates using any microbial oil which provides a sufficient amount of ARA to overcome the negative effects of dietary EPA. In particular, the ARA-containing oil will provide an ARA:EPA ratio of at least about 5:1 in the infant formula. More preferably, the ratio will be at least about 10:1 and, most preferably, it will be at least about 20:1. As can be seen, the higher the amount of ARA in the end product, with respect to the amount of EPA, the more desirable is the result.

In a process described herein, the fungi are cultivated under suitable ARA-containing oil producing cultivating conditions. In general, techniques of fungal cultivation are well known to those of skill in the art and those techniques can be applied to the process as described herein. For example, cultivation of an inoculating amount of fungus can occur in submerged culture in shake flasks. The flask is provided with a growth medium, seeded with fungal mycelium, and grown on a reciprocating shaker for about three to four days.

The composition of the growth medium can vary but always contains carbon and nitrogen sources. A preferred carbon source is glucose, amounts of which can range from about 10-100 grams glucose per liter of growth medium. Typically about 15 grams/liter are utilized for shaker flask culture. The amount can be varied depending upon the desired density of the final culture. Other carbon sources which can be used include molasses, high fructose corn syrup, hydrolyzed starch or any other low cost conventional carbon source used in fermentation processes. Additionally, lactose can be provided as a carbon source for *P*. *insidiosum.* Thus, whey permeate, which is high in lactose and is a very low cost carbon source, can be used as a substrate. Suitable amounts of these carbon sources can readily be determined by those of skill in the art. Usually, additional carbon needs to be added during the course of the cultivation. This is because the organisms use so much carbon that adding it all in a batch mode could prove unwieldy.

Nitrogen typically is provided in the form of yeast extract at a concentration of from about 2 to about 15 grams extract per liter of growth medium. Preferably, about four grams per liter are provided. Other nitrogen sources can be used, including peptone, tryptone, cornsteep liquor, etc. The amount to be added of these sources can easily be determined by those of skill in the art. Nitrogen can be added in a batch mode, i.e. all at one time prior to cultivation.

After cultivation for 3-4 days at a suitable temperature, typically about 25-30°C, an amount of fungi has grown which is sufficient for use as an inoculum in a conventional stirred tank fermentor (STF). Such fermentors are known to those of skill in the art and are commercially available. Fermentation can be carried out in batch, fed-batch, or continuous fermentation modes. Preferably, the STF is equipped with a Rushton-type turbine impeller.

The fermentor is prepared by adding the desired carbon and nitrogen sources. For example, a 1.5 liter fermentor can be prepared by mixing about 50 grams of glucose and about 15 grams of yeast extract per liter of tap water. As previously discussed, other carbon or nitrogen sources or mixtures thereof can be used.

The reactor containing the nutrient solution should be sterilized by, for example, heating prior to inoculation. After cooling to about 30°C, the inoculum can be added, and cultivation initiated. Gas exchange is provided by air sparging. The air sparging rate can vary, but preferably is adjusted to from about 0.5 to about 4.0 VVM (volume of air per volume of fermentor per minute). Preferably the dissolved oxygen level is kept at from about 10% to about 50% of the air saturation value of the solution. Accordingly, adjustments in the sparge rate may be required during cultivation. Agitation is desirable. The agitation is provided by the impeller. Agitation tip speed preferably is set within the range of from about 50 cm/sec to about 500 cm/sec, preferably from about 100 to 200 cm/sec.

In general, the amount of inoculum can vary. Typically, from about 2% to about 10% by volume of inoculum can be used. Preferably, in a fermentor seed train about 5% by volume of inoculum can be used.

Nutrient levels should be monitored. When glucose levels drop below 5 g/l, additional glucose should be added. A typical cultivation cycle utilizes about 100 grams of glucose and about 15 grams of yeast extract per liter. It is desirable to deplete the nitrogen during the course of the cultivation as this enhances oil production by the fungi. This is especially true when *M. alpina* is used as the production organism.

Occasionally, the culture will produce an excessive quantity of foam. Optionally, an antifoaming agent, such as those known to those of skill in the art, e.g. Mazu 310^{®}, can be added to prevent foam.

The temperature of cultivation can vary. However, those fungi which produce both ARA and EPA tend to produce less EPA and more ARA when cultivated at higher temperatures. For example, when *Mortierella alpina* is cultivated at less than 18°C, it begins to produce EPA. Thus, it is preferable to maintain the temperature at a level which induces the preferential production of ARA. Suitable temperatures are typically from about 25°C to about 30°C.

Preferably, cultivation continues until a desired biomass density is achieved. A desirable biomass is about 25 g/l of the organism. Such a biomass typically is attained within 48-72 hours after inoculation. At this time, the organisms typically contain about 5-40% complex lipids, i.e. oil, of which about 10-40% is ARA, and can be harvested.

Harvesting can be done by any suitable method such as, for example, filtration, centrifugation, or spray drying. Because of lower cost, filtration may be preferred.

After harvesting, the mycelial cake can be extracted. The mycelial cake refers to the collection of biomass resulting after harvest. The cake can be loose or pressed, crumbled or uncrumbled. Optionally, the cake can have any residual water removed, as by vacuum drying or lyophilization, prior to extraction. If this option is selected, it is preferable to use nonpolar solvents to extract the ARA-containing oil. While any non-polar extract is suitable, hexane is preferred.

Alternatively, the wet cake (which typically contains about 30-50% solids) can be crumbled and extracted directly using polar solvents such as ethanol or isopropyl alcohol, or supercritical fluid extraction with solvents such as CO₂ or NO. Preferably, the cakes are crumbled prior to extraction. Advantageously, the present invention permits the economical use of supercritical fluid extraction techniques. McHugh, et al., Supercritical Fluid Extraction, Butterworth (1986).

Such techniques are known to those of skill in the art and include those presently applied, for example, to decaffeinate coffee beans. While the yields from both wet and dry extractions are similar, wet extraction generally is a more economical process.

A preferable method of aqueous extraction involves mixing the mycelial biomass with the polar solvent isopropyl alcohol in a suitable reaction kettle. Such kettles are known. The use of three to six parts of solvent per part of biomass is desired. Most preferably, the mixing is done under nitrogen or in the presence of antioxidants to prevent the oxidation of the ARA in the lipid extract. As used herein "lipid extract", "oil", "lipid complex" and "fungal oil" are used interchangeably.

After extracting, the mixture can be filtered to remove the biomass from the solvent containing the lipid extract. At this point, the biomass can be recovered and used as a food supplement. As used herein, "food supplement" means feed or an additive to be mixed with typical feed, such as grain, etc., that can be provided to animals.

The solvent is separated from the lipid extract and also can be recovered for reuse, as by evaporation into a suitable collector, leaving what is referred to herein as the "crude oil." Use of isopropyl alcohol as the solvent desirably results in the removal of any residual water from the crude oil, as the evaporation removes the water/isopropyl alcohol azeotrope which has spontaneously formed.

While the crude oil can be used without further treatment, it also can be further purified. Processes such as those used in the preparation of lecithin from vegetable products, and known to those of skill in the art, can be used in this additional purification step. Such processes do not chemically or covalently modify the ARA-containing lipids or the ARA itself.

Yields vary, but typically are about 5 grams of ARA-containing phospholipid per 100 grams of dried mycelia. In the case of *M. alpina,* an additional 10-30 grams of triglyceride per 100 grams of dry mycelia can be obtained. Either the crude oil or the refined product can be used for administration to humans. Both shall be included within the definition of ARASCO as used herein.

The present invention provides human infant formulas, wherein the concentration of ARA in such formula closely approximates the concentration of ARA in human breast milk. Table 2 compares the composition of the fatty acids in ARASCO with those in breast milk and infant formula lacking and containing ARASCO.

**Table 2. Fatty acid composition of fungal oil products and mother's milk**

| Fatty Acid | ARASCO | Infant formula¹ | formula + oil | breast milk² |
|---|---|---|---|---|
| 8:0 | -- | 24.1 | 23.6 | 0.35 |
| 10:0 | -- | 17.7 | 17.3 | 1.39 |
| 12:0 | -- | 14.9 | 14.6 | 6.99 |
| 14:0 | 4.6 | 5.8 | 5.8 | 7.96 |
| 16:0 | 16.0 | 6.8 | 7.0 | 19.80 |
| 16:1 | 3.2 | 0.2 | 0.3 | 3.20 |
| 18:0 | -- | 2.3 | 2.3 | 5.91 |
| 19:1 | 26.4 | 10.0 | 10.3 | 34.82 |
| 15:2n6 | 9.9 | 17.4 | 17.3 | 16.00 |
| 18:3n3 | 4.1 | 0.9 | 1.0 | 0.62 |
| 20:1 | 2.2 | 0.1 | 0.14 | 1.10 |
| 20:2n6 | -- | -- | -- | 0.61 |
| 20:3n6 | 1.4 | -- | 0.03 | 0.42 |
| 20:4n6 | 32.0 | -- | 0.64 | 0.59 |
| 20:5n3 | -- | -- | -- | 0.03 |
| 22:1 | -- | -- | -- | 0.10 |
| 22:4n6 | -- | -- | -- | 0.21 |
| 22:5n6 | -- | -- | -- | 0.22 |
| 22:6n3 | -- | -- | -- | 0.19 |

| | | | | |
|---|---|---|---|---|
| ¹Simopculis, A., Omega-3 Fatty acids in Health and Disease, pp. 115-156 (1990) | | | | |

As can be seen, the amount of ARA present in the infant formula supplemented by ARASCO closely approximates the ARA levels in human breast milk. Additionally, the total fatty acid composition of the infant formula has not been significantly altered by the addition of the ARASCO. Between about 50 to about 1000 mg of ARASCO per liter of infant formula is used. The specific amount of ARASCO required depends upon the ARA content. This varies from about 10 to about 50% of the fatty acids in the oil. However, typically the ARA content is about 30%. When the ARA content is about 30%, an especially preferred supplementation rate is about 600 to 700 mg of ARASCO per liter of infant formula. Such a rate dilutes the pre-existing fat components of an infant formula such as Similac^{®} (Ross Laboratories, Columbus, Ohio) by only one part ARASCO to fifty parts formula oils. Preferably, EPA is present in the ARASCO in less than one fifth of the amount of ARA in the oil.

When *Pythium insidiosum* is used in the described process, the extracted ARA-containing oil is predominantly phospholipid. When *Mortierella alpina* is used in this process, the ARA-containing oil is predominantly triglyceride. Both forms of ARASCO are useful as additives to infant formula. The former not only provides the formula with ARA, but also with an emulsifier, i.e., phosphatidyl choline, which is commonly added to commercial formulas. The oil from *M. alpina* is likely to be more economical to produce.

The ARASCO used in the infant formula of the present invention is predominantly triglyceride.

The invention having been generally described, the following specific non-limiting examples are set forth to further illustrate the invention.

### Example 1. Preparation of P. insidiosum lipid and addition to infant formula

In an 80 liter (gross volume) fermentor, 51 liters of tap water, 1.2 kg glucose, 240 grams of yeast extract and 15 ml of MAZU 210S^{®} antifoam were combined. The fermentor was sterilized at 121°C for 45 minutes. An additional 5 liters of condensate water were added during the sterilization process. The pH was adjusted to 6.2, and approximately 1 liter of inoculum (at a cell density of 5-10g/l) of Pythium insidiosum (ATCC #28251) then was added. The agitation rate was adjusted to 125 RPM (250 cm/sec tip speed) and the aeration rate was set at 1 SCMF (standard cubic feet per minute). At hour 24 in the operation the aeration rate was increased to 3 SCFM. At hour 28 an additional 2 liters of 50% glucose syrup (1 kg glucose) were added. At hour 50 the fermentor was harvested, resulting in a yield of about 2.2 kg wet weight (approximately 15 g dry weight) per liter. Harvested biomass was squeezed to a high solids cake (50% solids) on a suction filter before freeze drying. The dried biomass was ground with a mortar and pestle and extracted with 1 liter of hexane per 200 grams of dry biomass at room temperature under continuous stirring for 2 hours. The mixture then was filtered and the filtrate evaporated to yield about 5-6 grams of crude oil per 100 grams of dry biomass. The biomass then was reextracted with 1 liter of ethanol per 20 grams of dry biomass for 1 hour at room temperature, filtered, and the solvent evaporated yielding an additional 22 grams of crude oil per 100 grams of dry biomass. The second fraction was predominantly phospholipids whereas the first fraction contained a mixture of phospholipids and triglycerides. The combined fractions produced an oil containing about 30-35% arachidonic acid and no detectable EPA. This oil was added dropwise to the commercial infant formula product Simulac^{®} (Ross Laboratories, Columbus, Ohio) at a supplementation rate of 600 mg per liter of prepared medium.

### Example 2. Preparation of M. alpina lipid and addition to infant formula

*Mortierella alpina* (ATCC #42430) was grown in a 2 liter shake flask containing 1 liter of tap water and 20 grams of potato dextrose medium. The flask was under constant orbital agitation and was maintained at 25°C for seven days. After harvesting by centrifugation, the biomass was freeze dried yielding about 8 grams of lipid-rich mycelia. The mycelia was extracted using hexane as in example #1 and about 2.4g of crude oil resulted. This oil contains about 23% arachidonic acid and was added to the commercial formula Similac^{®} dropwise in concentrations of 1000 mg per liter.

## Claims

1. Infant formula comprising between 50 to 1000 mg per liter of infant formula of an arachidonic acid (ARA) containing fungal oil (ARASCO), wherein
(i) EPA is present in the oil in less than one fifth of the amount of ARA in the oil,
(ii) the ARA content in the oil is from 10 to 50% of the fatty acids in the oil,
(iii) the oil is predominantly triglyceride and unmodified; and
(iv) the ARA:EPA ratio in the infant formula is at least 5:1.

2. Infant formula according to claim 1, wherein the ARA:EPA ratio in the infant formula is at least 10:1.

3. Infant formula according to claim 1, wherein the ARA:EPA ratio in the infant formula is at least 20:1.

4. Infant formula according to any of claims 1 to 3, wherein the ARASCO is refined.

5. Infant formula according to any preceding claim, wherein the ARASCO is obtainable by cultivating fungi under oil producing conditions in a growth medium, harvesting said fungi, extracting said oil from said harvested fungi and recovering the ARASCO.

6. Infant formula according to claim 5, wherein the ARASCO has been produced by *Mortierella alpina* and wherein the *Mortierella lpina* is cultivated at from 25°C to 30°C.

7. Infant formula according to any one of claims 5 to 6, wherein the harvesting results in a mycelial cake, and wherein the cake has any residual water removed prior to extraction.

8. Infant formula according to claim 7, wherein a non-polar solvent is used to extract the ARASCO.

9. Infant formula according to claim 8, wherein the non-polar solvent is hexane.

10. Infant formula according to any one of claims 5 to 9, wherein the harvesting results in a mycelial cake and wherein the cake is loose or pressed, crumbled or uncrumbled.

11. Infant formula according to any one of claims 5 to 10, wherein the growth medium contains glucose, molasses, high fructose corn syrup or hydrolyzed starch.

12. Infant formula according to any one of claims 5 to 11, wherein the growth medium contains 10 to 100 grams of glucose per liter of growth medium.

13. Infant formula according to any one of claims 5 to 12, wherein carbon is added during the course of the cultivation.

14. Infant formula according to any one of claims 5 to 13, wherein the growth medium contains peptone, tryptone or cornsteep liquor.

15. Infant formula according to any one of claims 5 to 14, wherein the growth medium contains yeast extract at a concentration of from 2 to 15 grams per liter of growth medium.

16. Infant formula according to any one of claims 5 to 15, wherein the nitrogen is depleted during the course of cultivation.

17. Infant formula according to any one of claims 5 to 16, wherein agitation is provided by an impeller.

## Patentansprüche

1. Säuglingsnahrung, die zwischen 50 und 1000 mg eines Arachidonsäure (ARA) enthaltenden Pilzöls (ARASCO) pro Liter Säuglingsnahrung umfasst, wobei
(i) EPA im öl in einer Menge vorhanden ist, welche weniger als ein Fünftel der Menge an ARA im öl beträgt;
(ii) der ARA-Gehalt im öl 10 bis 50% der Fettsäuren in dem öl beträgt;
(iii) das öl im Wesentlichen Triglycerid und unmodifiziert ist; und
(iv) das Verhältnis von ARA:EPA in der Säuglingsnahrung mindestens 5:1 beträgt.

2. Säuglingsnahrung nach Anspruch 1, wobei das ARA:EPA-Verhältnis in der Säuglingsnahrung mindestens 10:1 beträgt.

3. Säuglingsnahrung nach Anspruch 1, wobei das ARA:EPA-Verhältnis in der Säuglingsnahrung mindestens 20:1 beträgt.

4. Säuglingsnahrung nach einem der Ansprüche 1 bis 3, wobei das ARASCO raffiniert ist.

5. Säuglingsnahrung nach einem der vorhergehenden Ansprüche, wobei das ARASCO erhältlich ist, indem man Pilze unter ölproduzierenden Bedingungen in einem Wachstumsmedium kultiviert, die Pilze erntet, das öl von den geernteten pilzen extrahiert und das ARASCO gewinnt.

6. Säuglingsnahrung nach Anspruch 5, wobei das ARASCO von *Mortierella alpina* hergestellt wurde und wobei *Mortierella alpina* bei 25-30°C kultiviert wird.

7. Säuglingsnahrung nach einem der Ansprüche 5-6 wobei das Ernten zu einem Myzel-Kuchen führt, und wobei jeglicher Rest an Wasser vor der Extraktion aus dem Kuchen entfernt wurde.

8. Säuglingsnahrung nach Anspruch 7, wobei ein unpolares Lösungsmittel für die Extraktion von ARASCO verwendet wird.

9. Säuglingsnahrung nach Anspruch 8, wobei das unpolare Lösungsmittel Hexan ist.

10. Säuglingsnahrung nach einem der Ansprüche 5 bis 9, wobei das Ernten zu einem Myzel-Kuchen führt, und wobei der Kuchen lose oder gepresst, zerbröckelt oder nicht zerbröckelt ist.

11. Säuglingsnahrung nach einem der Ansprüche 5 bis 10, wobei das Wachstumsmedium Glukose, Melasse, Maissirup mit hohem Fruktose-Anteil oder hydrolysierte Stärke umfasst.

12. Säuglingsnahrung nach einem der Ansprüche 5 bis 11, wobei das Wachstumsmedium 10 bis 100 Gramm Glukose pro Liter Wachstumsmedium umfasst.

13. Säuglingsnahrung nach einem der Ansprüche 5 bis 12, wobei im Verlauf der Kultivierung Kohlenstoff zugegeben wird.

14. Säuglingsnahrung nach einem der Ansprüche 5 bis 13, wobei das Wachstumsmedium Pepton, Trypton oder Cornsteep-Flüssigkeit umfasst.

15. Säuglingsnahrung nach einem der Ansprüche 5 bis 14, wobei das Wachstumsmedium Hefeextrakt in einer Konzentration von 2 bis 15 Gramm pro Liter Wachstumsmedium umfasst.

16. Säuglingsnahrung nach einem der Ansprüche 5 bis 15, wobei der Stickstoff im Verlaufe der Kultivierung verbraucht wird.

17. Säuglingsnahrung nach einem der Ansprüche 5 bis 16, wobei Bewegung durch einen Rührer bereitgestellt wird.

## Revendications

1. Formule pour nourrissons comprenant entre 50 et 1 000 mg par litre de formule pour nourrissons d'une huile fongique (ARASCO) contenant de l'acide arachidonique (ARA), dans laquelle ;
(i) de l'EPA est présent dans l'huile en moins d'un cinquième de la quantité d'ARA dans l'huile,
(ii) la teneur en ARA dans l'huile est de 10 à 50 % des acides gras dans l'huile,
(iii) l'huile est essentiellement triglycéride et non modifiée ; et
(iv) le rapport ARA:EPA dans la formule pour nourrissons est d'au moins 5:1.

2. Formule pour nourrissons selon la revendication 1, dans laquelle le rapport ARA:EPA dans la formule pour nourrissons est d'au moins 10:1.

3. Formule pour nourrissons selon la revendication 1, dans laquelle le rapport ARA:EPA dans la formule pour nourrissons est d'au moins 20:1.

4. Formule pour nourrissons selon l'une quelconque des revendications 1 à 3, dans laquelle l'ARASCO est raffinée.

5. Formule pour nourrissons selon l'une quelconque des revendications précédentes, dans laquelle l'ARASCO peut être obtenue par culture de champignons dans des conditions de production d'huile dans un milieu de croissance, récolte desdits champignons, extraction de ladite huile à partir desdits champignons récoltés et récupération de l'ARASCO.

6. Formule pour nourrissons selon la revendication 5, dans laquelle l'ARASCO a été produite par *Mortierella alpina* et dans laquelle le *Mortierella alpina* est mis en culture entre 25 °C et 30 °C.

7. Formule pour nourrissons selon l'une quelconque des revendications 5 à 6, dans laquelle la récolte donne un gâteau mycélien, et dans laquelle le gâteau a été débarrassé de toute l'eau résiduelle avant l'extraction.

8. Formule pour nourrissons selon la revendication 7, dans laquelle un solvant apolaire est utilisé pour extraire l'ARASCO.

9. Formule pour nourrissons selon la revendication 8, dans laquelle le solvant apolaire est l'hexane.

10. Formule pour nourrissons selon l'une quelconque des revendications 5 à 9, dans laquelle la récolte donne un gâteau mycélien, et dans laquelle le gâteau est mou ou comprimé, émietté ou solide.

11. Formule pour nourrissons selon l'une quelconque des revendications 5 à 10, dans laquelle le milieu de croissance contient du glucose, de la mélasse, du sirop de glucose à haute teneur en fructose ou de l'amidon hydrolysé.

12. Formule pour nourrissons selon l'une quelconque des revendications 5 à 11, dans laquelle le milieu de croissance contient 10 à 100 g de glucose par litre de milieu de croissance.

13. Formule pour nourrissons selon l'une quelconque des revendications 5 à 12, dans laquelle le carbone est ajouté au cours de la culture.

14. Formule pour nourrissons selon l'une quelconque des revendications 5 à 13, dans laquelle le milieu de croissance contient une peptone, une peptone trypsique ou un extrait soluble de maïs.

15. Formule pour nourrissons selon l'une quelconque des revendications 5 à 14, dans laquelle le milieu de croissance contient un extrait de levure à une concentration de 2 à 15 g par litre de milieu de croissance.

16. Formule pour nourrissons selon l'une quelconque des revendications 5 à 15, dans laquelle l'azote est appauvri au cours de la culture.

17. Formule pour nourrissons selon l'une quelconque des revendications 5 à 16, dans laquelle l'agitation est assurée par un agitateur.
